# EUROPEAN PATENT APPLICATION

(11) **EP 3 510 923 A1**
(43) Date of publication of application: **17.07.2019**
(21) Application number: 17848873.0
(22) Date of filing: 08.09.2017
(51) Int. Cl.: A61B 5/0484, A61B 5/0476

(54) **BRAIN OSCILLATION FREQUENCY MODULATION DEVICE**

(30) Priority: 09.09.2016 JP 2016176669
(71) Applicant: National Institute of Information and Communications Technology, Tokyo 184-8795 (JP)
(72) Inventor: AMANO Kaoru, Koganei-shi Tokyo 184-8795 (JP)
(74) Representative: EP&C
(86) International application number: PCT/JP2017/032418
(87) International publication number: WO 2018/047935

(57) **Abstract**

This neural oscillation frequency modulation device changes a frequency of neural oscillation and includes an amplitude modulation unit which generates a modulation signal obtained by amplitude-modulating a carrier signal having a higher frequency than a target frequency band of the neural oscillation on the basis of a prescribed frequency in the target frequency band and an electric stimulation output unit which outputs, as an electric stimulus, an electric stimulus signal based on the modulation signal generated by the amplitude modulation unit to an electrode unit connectable to the head of a subject.

## Description

### TECHNICAL FIELD

The present invention relates to a neural oscillation frequency modulation device.

Priority is claimed on Japanese Patent Application No. 2016-176669, filed September 9, 2016, the content of which is incorporated herein by reference.

### BACKGROUND ART

In recent years, in order to change neural oscillation (for example, brain waves such as alpha waves), a method of providing an alternating current (AC) electric stimulus of a desired frequency to the brain has been proposed (for example, refer to Non-Patent Document 1). Attempts have been performed to utilize this method to check the influence on cognitive functions and perceptual functions of the brain, for example, by changing a frequency of brain waves.

### [Documents of the Prior Art]

### [Patent Document]

[Non-Patent Document 1]
Roberto C, Geraint R, Vincenzo R, "Individual Differences in Alpha Frequency Drive Crossmodal Illusory Perception," Current Biology 25, 231 to 235, January 19, 2015

### SUMMARY OF INVENTION

### [Problems to be Solved by the Invention]

However, in the above-described related art, artifacts (noise) due to an AC electric stimulus are so large. Thus, there is a problem that it is not possible to measure the brain activity of the target frequency band of the AC electric stimulus using an electroencephalogram (EEG), a magnetoencephalogram (MEG), or the like. That is to say, in the above-described related art, there is a problem that it cannot be checked whether the neural oscillation is actually changed to the target frequency using the electroencephalography (EEG) or the magnetoencephalography (MEG).

The present invention was made to solve the above-described problem, and an objective of the present invention is to provide a neural oscillation frequency modulation device capable of checking a changed frequency of neural oscillation.

### [Means for Solving the Problems]

In order to solve the above-described problems, an aspect of the present invention is a neural oscillation frequency modulation device which changes a frequency of neural oscillation including: an amplitude modulation unit which generates a modulation signal obtained by amplitude-modulating a carrier signal of a higher frequency than the target frequency band of the neural oscillation on the basis of a prescribed frequency in the target frequency band; and an electric stimulation output unit which outputs, as an electrical stimulus, an electric stimulus signal based on the modulation signal generated by the amplitude modulation unit to an electrode unit connectable to the head of a subject.

Also, in an aspect of the present invention, the electric stimulation output unit may generate, as the electric stimulation signal, a signal obtained by converting a maximum amplitude value of the modulation signal into a prescribed current value on the basis of the modulation signal and output the generated electric stimulus signal.

In an aspect of the present invention, the neural oscillation frequency modulation device may include a correcting unit which corrects the electric stimulus signal so that the neural oscillation frequency measured in the subject coincides with the prescribed frequency.

In an aspect of the present invention, in the neural oscillation frequency modulation device, the correcting unit may correct a modulation frequency at which the modulation signal is amplitude-modulated so that the neural oscillation frequency coincides with the prescribed frequency.

### [Advantageous Effects of the Invention]

According to the present invention, it is possible to check a changed frequency of neural oscillation.

### BRIEF DESCRIPTION OF DRAWINGS

Fig. 1 is a functional block diagram illustrating an example of a brain activity measuring system according to a first embodiment.
Fig. 2 is a diagram illustrating an example of a current stimulus signal associated with the first embodiment.
Fig. 3 is a diagram illustrating an effect of a neural oscillation frequency modulation device according to the first embodiment.
Fig. 4 is a functional block diagram illustrating an example of a brain activity measuring system according to a second embodiment.

### EMBODIMENTS FOR CARRYING OUT THE INVENTION

A neural oscillation frequency modulation device according to an embodiment of the present invention will be described below with reference to the drawings.

### [First embodiment]

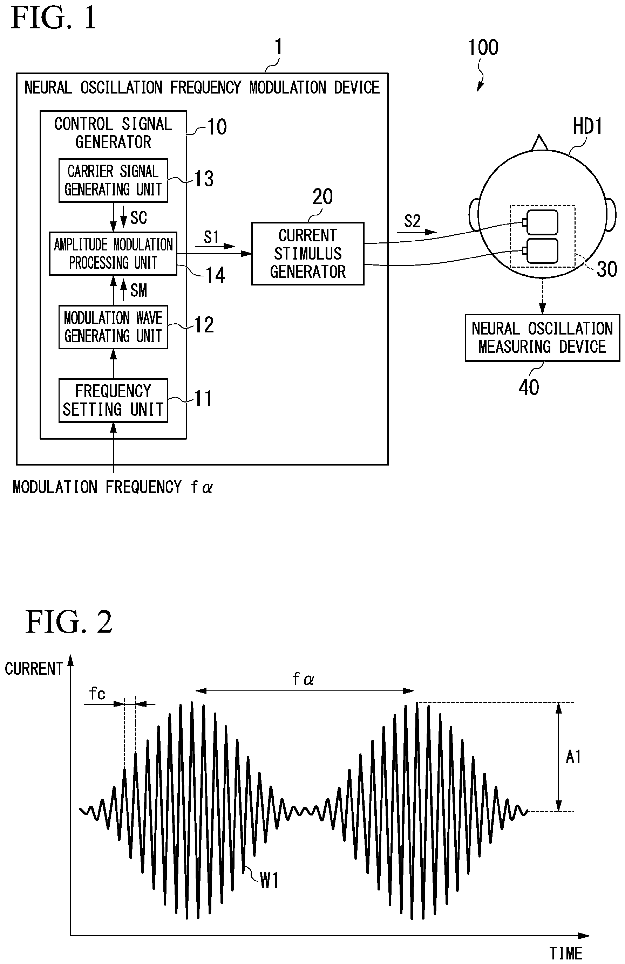

Fig. 1 is a functional block diagram illustrating an example of a brain activity measuring system 100 according to a first embodiment.

As illustrated in Fig. 1, the brain activity measuring system 100 includes a neural oscillation frequency modulation device 1 and a neural oscillation measuring device 40.

The neural oscillation frequency modulation device 1 outputs an electric stimulus signal (for example, an alternating current (AC) current stimulus signal) to an electrode unit 30 connected to the head HD1 of a subject and changes a frequency of neural oscillation (for example, alpha waves). For example, the neural oscillation frequency modulation device 1 outputs a current stimulus signal S2 to the head HD1 of the subject via the electrode unit 30 on the basis of a value of an input modulation frequency fα (an example of a prescribed frequency) and changes a frequency of neural oscillation of the subject to the input modulation frequency fα.

Here, neural oscillation is a periodic brain activity, and for example, is brain waves or the like such as alpha waves or beta waves. It should be noted that, in this embodiment, as an example of the neural oscillation, a case in which a frequency of alpha waves is changed will be described.

Alpha waves are, for example, components of 8 hertz (Hz) to 13 Hz among brain waves generated in the brain. In the following description, in this embodiment, description will be provided assuming that a target frequency band which is a frequency band to be changed to in the neural oscillation frequency modulation device 1 is 8 Hz to 13 Hz.

Also, the neural oscillation frequency modulation device 1 includes a control signal generator 10 and a current stimulus generator 20.

The control signal generator 10 (an example of an amplitude modulation unit) generates a control signal S1 (a modulation signal) obtained by amplitude-modulating (AM: Amplitude Modulation) a carrier signal SC having a higher frequency (for example, 200 Hz) than a target frequency band of the neural oscillation on the basis of a prescribed frequency in the target frequency band. It should be noted that, as described above, the target frequency band of the neural oscillation herein is an alpha wave frequency band (8 Hz to 13 Hz). Furthermore, a prescribed frequency (a modulation frequency fα) is, for example, a frequency between 8 Hz and 13 Hz.

Also, the control signal generator 10 includes a frequency setting unit 11, a modulation wave generating unit 12, a carrier signal generating unit 13, and an amplitude modulation processing unit 14.

The frequency setting unit 11 acquires frequency information indicating the value of the input modulation frequency fα, sets (holds) the acquired frequency information, and outputs the frequency information to the modulation wave generating unit 12. It should be noted that the value of the modulation frequency fα may be set on the basis of the alpha wave frequency of the subject in a state in which current stimulation is not provided or may be set irrespective of the alpha wave frequency of the subject. For example, the value of the modulation frequency fα may be set to ±1 Hz, ±2 Hz, or the like as the alpha wave frequency of the subject in a state in which current stimulation is not provided.

The modulation wave generating unit 12 acquires the frequency information (fα) set in the frequency setting unit 11 and generates, for example, a modulation wave signal SM of a frequency fα on the basis of the acquired frequency information. Here, the modulation frequency fα is a frequency in the alpha wave frequency band (8 Hz to 13 Hz) and the modulation wave signal SM is, for example, a sine wave signal of a frequency between 8 Hz and 13 Hz. The modulation wave generating unit 12 outputs the generated modulation wave signal SM to the amplitude modulation processing unit 14.

The carrier signal generating unit 13 is, for example, an oscillation circuit and generates the carrier signal SC.

Here, the carrier signal SC is a signal having a higher frequency than the alpha wave frequency band (8 Hz to 13 Hz), for example, a sine wave signal (a sin wave signal) of 200 Hz that does not affect the alpha wave measurement. The carrier signal generating unit 13 outputs the generated carrier signal SC to the amplitude modulation processing unit 14.

The amplitude modulation processing unit 14 generates the control signal S1 (a modulation signal) obtained by amplitude-modulating the carrier signal SC generated by the control signal generator 10 on the basis of the modulation wave signal SM generated by the modulation wave generating unit 12. Here, the control signal S1 is, for example, a modulation signal in which the amplitude of the carrier signal SC of 200 Hz is modulated by a sine wave of the frequency fα (a prescribed frequency) of the modulation wave signal SM. For example, the amplitude modulation processing unit 14 generates, as the control signal S1, an amplitude modulation signal whose maximum amplitude is a prescribed voltage. Furthermore, the amplitude modulation processing unit 14 outputs the generated control signal S1 to the current stimulus generator 20.

The current stimulus generator 20 (an example of an electric stimulation output unit) is, for example, a transcranial AC stimulation device and outputs the current stimulus signal S2 (an example of an electric stimulus signal) based on the control signal S1 generated by the control signal generator 10 to the electrode unit 30. For example, the current stimulus generator 20 converts the control signal S1 amplitude-modulated using the modulation frequency fα so that a prescribed voltage has a maximum amplitude into the current stimulus signal S2 in which a current is amplitude-modulated using the modulation frequency fα so that a prescribed current value (for example, 1 milliampere (mA)) has a maximum amplitude. That is to say, the current stimulus generator 20 generates the current stimulus signal S2, for example, like a waveform W1 illustrated in Fig. 2 on the basis of the control signal S1.

Fig. 2 is a diagram illustrating an example of a current stimulus signal associated with this embodiment.

In Fig. 2, a horizontal axis indicates time and a vertical axis indicates a current. Furthermore, the waveform W1 illustrates an example of the above-described current stimulus signal S2.

Also, a carrier frequency fc (a transmission wave frequency) of the current stimulus signal S2 is a frequency of the carrier signal SC and is, for example, 200 Hz. Furthermore, the modulation frequency fα of the current stimulus signal S2 is a frequency input to the modulation wave generating unit 12 and is a frequency of the modulation wave signal SM. Moreover, a maximum amplitude value A1 of the current stimulus signal S2 is, for example, 1 mA. When the current stimulus signal S2 is applied to the head HD1 of the subject, for example, the maximum amplitude value A1 is determined so that it does not harm the subject's health.

It should be noted that the waveform of the control signal S1 generated by the control signal generator 10 is the same as the waveform W1 illustrated in Fig. 2, except that an amplitude is a voltage.

The current stimulus generator 20 generates the current stimulus signal S2 in which a current is amplitude-modulated on the basis of the control signal S1 in which a voltage is amplitude-modulated and outputs, as an electric stimulus, the generated current stimulus signal S2 to the electrode unit 30. In this way, the current stimulus generator 20 generates, as the current stimulus signal S2, a signal obtained by converting a maximum amplitude value of the control signal S1 into a prescribed current value (for example, 1 mA) on the basis of the control signal S1 and outputs the generated current stimulus signal S2 to the electrode unit 30.

The electrode unit 30 is, for example, an electrode on the scalp connectable to the head of the subject (the head HD1 of the subject). The electrode unit 30 is electrically connected to the head HD1 of the subject and provides electrical stimulation (for example, current stimulation) due to the current stimulus signal S2 generated by the current stimulus generator 20 to the head HD1 of the subject.

The neural oscillation measuring device 40 measures neural oscillation (for example, alpha waves), and for example, an electroencephalography (EEG) which measures the electrical activity of the brain, a magnetoencephalography (MEG) which measures a weak magnetic field generated by the electrical activity of the brain, and the like. The neural oscillation measuring device 40 measures the frequency of the neural oscillation (for example, alpha waves) in a state in which the current stimulus signal S2 is output from the neural oscillation frequency modulation device 1 to the electrode unit 30.

The neural oscillation measuring device 40 measures the alpha wave frequency of the subject, thereby checking that the alpha wave frequency has been changed (modulated) by the neural oscillation frequency modulation device 1.

An operation of the neural oscillation frequency modulation device 1 according to this embodiment will be described below.

In the neural oscillation frequency modulation device 1, for example, when information indicating a modulation frequency (fα=10 Hz) is input, the frequency setting unit 11 of the control signal generator 10 internally sets the information as frequency information and supplies the frequency information to the modulation wave generating unit 12. The modulation wave generating unit 12 acquires the frequency information (fα) set in the frequency setting unit 11 and generates the modulation wave signal SM of a modulation frequency (fα=10 Hz) on the basis of the acquired frequency information. The modulation wave generating unit 12 outputs the generated modulation wave signal SM of 10 Hz to the amplitude modulation processing unit 14.

Also, the carrier signal generating unit 13 generates the carrier signal SC of 200 Hz. The carrier signal generating unit 13 outputs the generated carrier signal SC to the amplitude modulation processing unit 14.

Subsequently, the amplitude modulation processing unit 14 generates the control signal S1 which has been amplitude-modulated using the modulation wave signal SM of 10 Hz into the carrier signal SC of 200 Hz. The carrier signal generating unit 13 outputs the generated control signal S1 to the current stimulus generator 20.

The current stimulus generator 20 generates a current stimulus signal S2 obtained by changing a maximum amplitude of the control signal S1 generated by the control signal generator 10 into, for example, 1 mA. That is to say, for example, the current stimulus generator 20 generates a current stimulus signal S2 which has a waveform like the waveform W1 in Fig. 2 and in which the modulation frequency fα is 10 Hz and the maximum amplitude value A1 of a current is 1 mA. The current stimulus generator 20 outputs the generated current stimulus signal S2 to the head HD1 of the subject via the electrode unit 30. Thus, the alpha waves of the subject are changed to 10 Hz which is a modulation frequency.

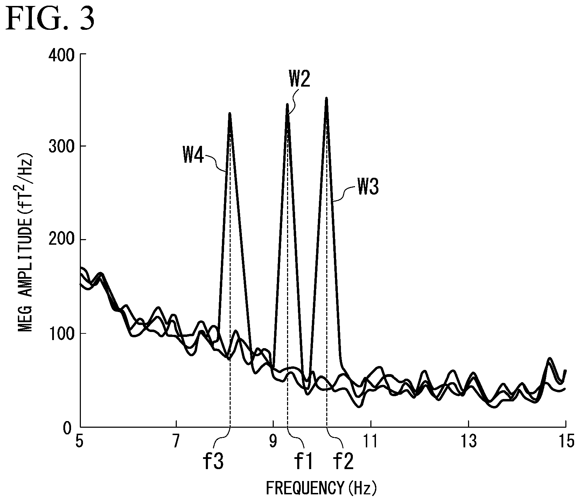

An effect of the neural oscillation frequency modulation device 1 according to this embodiment will be described below with reference to Fig. 3.

Fig. 3 is a diagram illustrating an effect of the neural oscillation frequency modulation device 1 according to this embodiment.

The graph illustrated in Fig. 3 shows a change in a magnetoencephalography (MEG) when current stimulation is provided to the head HD1 of the subject using the neural oscillation frequency modulation device 1. It should be noted that, in this graph, a vertical axis indicates an MEG amplitude and a horizontal axis indicates a frequency.

In the graph, a waveform W2 shows the measurement results of a magnetoencephalogram before current stimulation using the neural oscillation frequency modulation device 1 is provided. In the waveform W2, a frequency f1 indicating a peak value indicates an alpha wave frequency (PAF: peak alpha frequency) of the subject.

Also, a waveform W3 shows the measurement results of the electroencephalogram when current stimulation is provided using the neural oscillation frequency modulation device 1 at a modulation frequency of the frequency f1+1 Hz. In the waveform W3, a frequency f2 indicating a peak value indicates an alpha wave frequency of the subject to whom current stimulation has been provided and it is shown that the frequency f2 has increased by about 1 Hz with respect to the frequency f1 due to current stimulation using the neural oscillation frequency modulation device 1.

Also, a waveform W4 shows the measurement results of the electroencephalogram when current stimulation is provided using the neural oscillation frequency modulation device 1 at a modulation frequency of the frequency f1-1 Hz. In the waveform W4, a frequency f3 indicating a peak value indicates an alpha wave frequency of the subject to whom current stimulation has been provided and it is shown that the frequency f3 has decreased by about 1 Hz with respect to the frequency f1 due to current stimulation using the neural oscillation frequency modulation device 1.

In this way, the neural oscillation frequency modulation device 1 can change (modulate) the alpha wave frequency of the subject by changing the modulation frequency fα.

As described above, the neural oscillation frequency modulation device 1 according to this embodiment changes the frequency of the neural oscillation (for example, alpha waves) and includes the control signal generator 10 (an amplitude modulation unit) and the current stimulus generator 20 (an electric stimulation output unit). The control signal generator 10 generates the control signal S1 (a modulation signal) obtained by amplitude-modulating a carrier signal SC of a higher frequency (for example, 200 Hz) than the target frequency band of the neural oscillation (for example, 8 Hz to 13 Hz which are alpha wave frequencies) on the basis of a prescribed frequency (a modulation frequency fα) in the target frequency band. The current stimulus generator 20 (an electric stimulation output unit) outputs the current stimulus signal S2 (an electric stimulus signal; refer to the waveform W1 in Fig. 2) based on the control signal S1 (a modulation signal) generated by the control signal generator 10 to the electrode unit 30 connectable to the head of the subject (the head HD1 of the subject).

In this case, frequency components included in the current stimulus signal S2 are theoretically the frequency fc (the carrier frequency) of the carrier signal SC, a frequency (fc-fα) obtained by subtracting a prescribed frequency (a modulation frequency fα) from the frequency fc of the carrier signal SC, and a frequency (fc+fα) obtained by adding a prescribed frequency (a modulation frequency fα) to the frequency fc of the carrier signal SC. For example, when the frequency fc of the carrier signal SC is set to be 200 Hz and a prescribed frequency fα is set to be 10 Hz, the frequency components included in the current stimulus signal S2 are 200 Hz, 190 Hz, and 210 Hz.

In this way, since the frequency components included in the current stimulus signal S2 are frequencies away from frequencies of the neural oscillation (for example, alpha waves), the neural oscillation frequency modulation device 1 according to this embodiment can reduce artifacts (noise). Thus, the neural oscillation frequency modulation device 1 according to this embodiment can check the changed frequency of the neural oscillation (for example, alpha waves).

Also, since it is possible to check the frequency of the neural oscillation (for example, alpha waves) changed by the neural oscillation frequency modulation device 1, when a relationship between the frequency of the neural oscillation (for example, alpha waves) and the brain activity is analyzed, analysis can be accurately performed. For example, it is conceivable that the alpha wave frequency has an influence on cognitive functions, perceptual functions, and the like associated with information processing such as vision. For example, a relationship between the alpha wave frequency and cognitive functions and perceptual functions associated with information processing such as vision is analyzed, and thus the neural oscillation frequency modulation device 1 and the brain activity measuring system 100 according to this embodiment can check the alpha wave frequency, and it is possible to accurately analyze the relationship using the secured alpha wave frequency.

Also, in this embodiment, the current stimulus generator 20 generates a signal obtained by converting a maximum amplitude value of the control signal S1 into a prescribed current value (for example, 1 mA) as the current stimulus signal S2 on the basis of the control signal S1 and outputs the generated current stimulus signal S2. A prescribed current value (for example, 1 mA) is determined, for example, so that the current stimulus signal S2 does not harm the subject's health when it is applied to the head HD1 of the subject.

Thus, the neural oscillation frequency modulation device 1 according to this embodiment can change the frequency of the neural oscillation (for example, alpha waves) without impairing the subject's health.

### [Second embodiment]

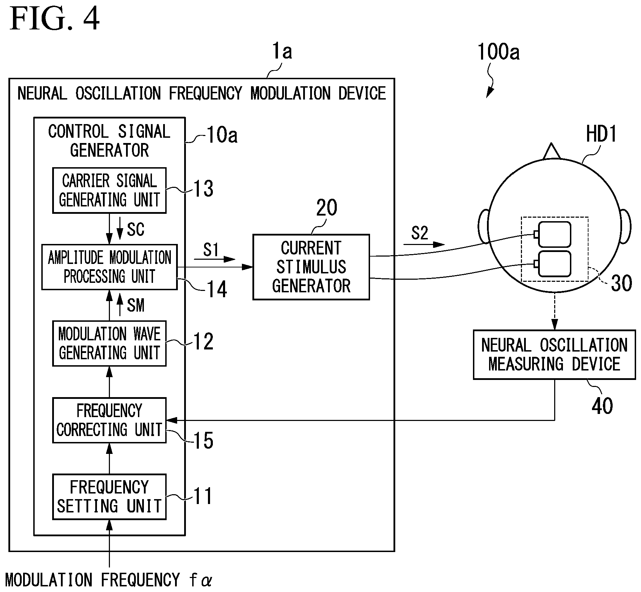

A neural oscillation frequency modulation device 1a according to a second embodiment will be described below with reference to Fig. 4.

Fig. 4 is a functional block diagram illustrating an example of a brain activity measuring system 100a according to the second embodiment.

As illustrated in Fig. 4, the brain activity measuring system 100a includes the neural oscillation frequency modulation device 1a and a neural oscillation measuring device 40.

The neural oscillation frequency modulation device 1a includes a control signal generator 10a and a current stimulus generator 20.

Also, the control signal generator 10a includes a frequency setting unit 11, a modulation wave generating unit 12, a carrier signal generating unit 13, an amplitude modulation processing unit 14, and a frequency correcting unit 15.

This embodiment and the first embodiment differ in that the control signal generator 10a in this embodiment includes the frequency correcting unit 15.

It should be noted that constituent elements of Fig. 4 that are the same as those of Fig. 1 will be denoted by the same reference numerals and description thereof will be omitted.

The frequency correcting unit 15 (an example of a correcting unit) corrects a current stimulus signal S2 so that a frequency of neural oscillation (for example, an alpha wave frequency) measured at the subject coincides with a prescribed frequency (a modulation frequency fα). For example, the frequency correcting unit 15 corrects a modulation frequency obtained by amplitude-modulating a modulation wave signal SM so that an alpha wave frequency measured by the neural oscillation measuring device 40 coincides with the modulation frequency fα set in the frequency setting unit 11. The frequency correcting unit 15 outputs the corrected modulation frequency to the modulation wave generating unit 12 as frequency information.

For example, when the alpha wave frequency measured by the neural oscillation measuring device 40 is higher than the modulation frequency fα set in the frequency setting unit 11, the frequency correcting unit 15 corrects frequency information to decrease and outputs the reduced frequency information to the modulation wave generating unit 12. Furthermore, for example, when the alpha wave frequency measured by the neural oscillation measuring device 40 is lower than the modulation frequency fα set in the frequency setting unit 11, the frequency correcting unit 15 corrects frequency information to increase and outputs the increased frequency information to the modulation wave generating unit 12. The frequency correcting unit 15 not only holds the corrected frequency information, but also holds and periodically corrects the corrected frequency information.

The modulation wave generating unit 12 acquires the corrected frequency information from the frequency correcting unit 15 and generates the modulation wave signal SM on the basis of the acquired frequency information. That is to say, the modulation wave generating unit 12 generates the modulation wave signal SM of a frequency indicated by the frequency information. The modulation wave generating unit 12 outputs the generated modulation wave signal SM to the amplitude modulation processing unit 14.

It should be noted that, since the processes of the carrier signal generating unit 13, the amplitude modulation processing unit 14, and the current stimulus generator 20 are the same as those in the first embodiment, description thereof will be omitted herein.

As described above, the neural oscillation frequency modulation device 1a according to this embodiment includes the control signal generator 10a (an amplitude modulation unit) and the current stimulus generator 20 (an electric stimulation output unit).

Thus, the neural oscillation frequency modulation device 1a according to this embodiment accomplishes the same effects as those of the first embodiment and can check the changed frequency of the neural oscillation (for example, an alpha wave).

Also, the neural oscillation frequency modulation device 1a according to this embodiment includes the frequency correcting unit 15 (a correcting unit). The frequency correcting unit 15 corrects the current stimulus signal S2 (an electric stimulus signal) so that the frequency of the neural oscillation (for example, an alpha wave) measured in the subject coincides with a prescribed frequency (a modulation frequency fα).

Thus, in the neural oscillation frequency modulation device 1a according to this embodiment, the current stimulus signal S2 is corrected on the basis of the measured frequency of the neural oscillation (for example, an alpha wave). Thus, it is possible to accurately change the frequency of the neural oscillation (for example, an alpha wave).

Also, in this embodiment, the frequency correcting unit 15 corrects a modulation frequency (frequency information) obtained by amplitude-modulating the control signal S1 (a modulation signal) so that the measured alpha wave frequency coincides with a prescribed frequency (a modulation frequency fα).

Thus, the neural oscillation frequency modulation device 1a according to this embodiment can generate an appropriate control signal S1 (a modulation signal) using a simple means of changing (correcting) a modulation frequency. Therefore, the neural oscillation frequency modulation device 1a according to this embodiment can change the frequency of the neural oscillation (for example, an alpha wave) with high accuracy using a simple means.

It should be noted that the present invention is not limited to the above-described embodiments and can be modified without departing from the gist of the present invention.

For example, although an example in which the neural oscillation frequency modulation device 1 (1a) changes an alpha wave frequency as an example of the neural oscillation has been described in the above-described embodiments, the neural oscillation frequency modulation device 1 (1a) may change frequencies of other brain waves such as theta waves, beta waves and gamma waves.

Also, although an example in which the neural oscillation frequency modulation device 1 (1a) generates the current stimulus signal S2 as an example of the electric stimulus signal has been described in the above-described embodiments, the present invention is not limited thereto. In addition, for example, other electric stimulus signals such as a voltage stimulus signal may be used as long as the electric stimulus signal is an electric stimulus signal obtained through amplitude-modulating using the modulation frequency fα.

Also, the constitution of the control signal generator 10 (10a) is not limited to the above-described embodiments, the control signal S1 may be generated through analog signal processing, and the control signal S1 may be generated through digital signal processing. That is to say, for example, the control signal generator 10 (10a) may generate the control signal S1 by converting waveform data of the control signal S1 generated through calculation processing into the control signal S1 through a digital to analog converter (DAC).

Also, in the above-described embodiments, the control signal generator 10 (10a) may modulate a plurality of frequencies of the neural oscillation such as simultaneous modulation of an alpha wave and a beta wave with a linear sum of amplitude modulation of 10 Hz to the carrier signal SC of 200 Hz and amplitude modulation of 20 Hz to the carrier signal SC of 300 Hz.

In addition, the control signal generator 10 (10a) may change a position in which the electrode unit 30 is installed in accordance with a position in which an alpha wave is generated (for example, the occipital region, the temporal region, the forehead head, and the like). Furthermore, in order to simultaneously modulate the alpha wave frequency at a plurality of positions, the control signal generator 10 (10a) may include a plurality of electrode units 30.

Also, although an example in which the neural oscillation frequency modulation device 1 (1a) is constituted of two devices, i.e., the control signal generator 10 (10a) and the current stimulus generator 20 has been described in the above-described embodiments, the control signal generator 10 (10a) may be configured to include the current stimulus generator 20 and the current stimulus generator 20 may be configured to include a part of the control signal generator 10 (10a) or the entire control signal generator 10 (10a). Furthermore, the neural oscillation frequency modulation device 1 (1a) may be configured as a separate device from the control signal generator 10 (10a) and the current stimulus generator 20.

The above-described brain activity measuring system 100 (100a) and neural oscillation frequency modulation device 1 (1a) include a computer system therein. Moreover, the process of generating the above-described electric stimulus signal is stored in a computer readable recording medium in the form of a program and the above processes are performed by the computer which has the program read therein and is executed through the program. Here, a computer readable recording medium refers to a magnetic disk, a magneto-optical disk, a compact disc (CD)-read only memory (ROM), a digital versatile disc (DVD)-ROM, a semiconductor memory, and the like. Furthermore, this computer program may be delivered to a computer through a communication circuit and the computer which receives this distribution may execute the program.

Also, a part or all of the above-described functions may be realized as an integrated circuit such as a large scale integration (LSI). The above-described functions may be individually implemented as a process and a part of all of the above-described functions may be integrated into a processor. Furthermore, a method of implementing a part or all of the functions as an integrated circuit is not limited to an LSI and may be realized by a dedicated circuit or a general purpose processor. In addition, when an integrated circuit technology to replace an LSI appears due to the advance in a semiconductor technology, an integrated circuit based on this technology may be used.

### [Reference Symbols]

- 1, 1a: Neural oscillation frequency modulation device
- 10, 10a: Control signal generator
- 11: Frequency setting unit
- 12: Modulation wave generating unit
- 13: Carrier signal generating unit
- 14: Amplitude modulation processing unit
- 15: Frequency correcting unit
- 20: Current stimulus generator
- 30: Electrode unit
- 40: Neural oscillation measuring device
- 100, 100a: Brain activity measuring system
- HD1: Head of subject

## Claims

1. A neural oscillation frequency modulation device which changes a frequency of neural oscillation, comprising:
an amplitude modulation unit which generates a modulation signal obtained by amplitude-modulating a carrier signal of a higher frequency than a target frequency band of the neural oscillation on the basis of a prescribed frequency in the target frequency band; and
an electric stimulation output unit which outputs an electric stimulus signal based on the modulation signal generated by the amplitude modulation unit to an electrode unit connectable to the head of a subject.

2. The neural oscillation frequency modulation device according to claim 1, wherein the electric stimulation output unit generates, as the electric stimulation signal, a signal obtained by converting a maximum amplitude value of the modulation signal into a prescribed current value on the basis of the modulation signal and outputs the generated electric stimulus signal.

3. The neural oscillation frequency modulation device according to claim 1 or 2, comprising:
a correcting unit which corrects the electric stimulus signal so that the neural oscillation frequency measured in the subject coincides with the prescribed frequency.

4. The neural oscillation frequency modulation device according to claim 3, wherein the correcting unit corrects a modulation frequency which amplitude-modulates the modulation signal so that the neural oscillation frequency coincides with the prescribed frequency.
